Europäisches Patentamt

European Patent Office

Office européen des brevets

(19)

(11) Publication number: **0 335 840**
**A2**

(12) **EUROPEAN PATENT APPLICATION**

(21) Application number: **89830133.8**

(22) Date of filing: **23.03.89**

(51) Int. Cl.⁴: **A 61 M 5/14**

(30) Priority: **30.03.88 IT 4780188**

(43) Date of publication of application:
**04.10.89 Bulletin 89/40**

(84) Designated Contracting States:
**CH DE ES FR GB LI**

(71) Applicant: **Barbabella, Urbano**
**5 Via Carlo Tivaroni**
**I-00143 Roma RM (IT)**

(72) Inventor: **Barbabella, Urbano**
**5 Via Carlo Tivaroni**
**I-00143 Roma RM (IT)**

(74) Representative: **Di Cerbo, Mario et al**
**Società Italiana Brevetti Piazza Poli, 42**
**I-00187 Roma RM (IT)**

(54) **A gravity valve device to prevent liquids from draining completely out of vessels.**

(57) The device prevents a liquid from emptying completely out of a higher positioned vessel, for example a phleboclysis flask. The device consists essentially of a chamber having two necks, substantially circular in section, on its opposite ends, and internal grooving at the base of the upper neck. The chamber may comprise two half-units that can be non-distructively disassembled. A cut-off and/or sealing element moves freely inside the chamber, being built of material such that it floats in the liquid intended to flow into the device, and shaped so as to lodge in the orifice of either the upper or the lower neck. A LED and a photodiode or photoresistor may be incorporated on opposite sides of the upper part of the chamber so as to generate an alarm signal when the cut-off element is in the lower part of the device.

FIG 1

## Description

## A GRAVITY VALVE DEVICE TO PREVENT LIQUIDS FROM DRAINING COMPLETELY OUT OF VESSELS

The present invention refers to a gravity valve device that prevents liquids from emptying out completely from a higher-placed vessel into its outflow tube. In particular, the subject matter of the invention is a device that can be used to cut off the flow from a phleboclysis flask automatically, in order to prevent coagulation in the intravenous needle and all the negative consequences that may result from said event.

The problem of preventing the complete draining of liquid from a vessel (for example, the liquid contained in a phleboclysis flask), and interrupting the outflow before the undesirable event occurs, has already been faced in the past.

In fact, the state of the art in this field provides pinch-cock type devices, consisting of adjustable plastic or metal clamps. These devices make it possible on the one hand to regulate the flow of liquid through the tube and, on the other hand, for an operator to stop the outflow when the liquid in the flask is about to drain out completely, and so prevent it from draining out from the tube as well.

The main drawback of the aforementioned devices is undoubtedly that they require an operator to be continually, or almost continually, present to check the level of the liquid and to close the pinch-cock valve at the right moment.

This disadvantage of the devices according to the prior art can be avoided with the device of the present invention, which has further advantages to be illustrated hereinafter.

The device according to the present invention for preventing vessels from draining completely out into their deflux tubes comprises essentially a chamber having two necks, substantially circular in section, on its opposite ends and an internal grooving at the orifice of the upper neck, and inside the central part of said chamber a freely moving cut-off and/or sealing element made of material such as to allow it to float in the liquid intended to flow through the device, and shaped so as to lodge in the orifice of either the upper or the lower neck. A LED and a photodiode or photoresistor may be incorporated on opposite sides of the upper portion of the central part of the device. As shall be shown hereinafter, this embodiment of the invention can function as a generator of an alarm signal when the cut-off element is in the lower portion of the central part of the chamber.

In both the aforesaid embodiments, the cut-off and/or sealing element of the device may be a hollow sphere whose diameter is greater than that of the section of the two necks located at the opposite ends of the chamber. Alternatively, for example, the cut-off and/or sealing element may consist of an upright bell whose dimensions, with respect to those of the chamber, are such that it cannot turn upsidedown inside the latter. In this case the bottom of the bell flairs outward in the form of a ring whose outer edge slopes downward.

One version of the device according to the present invention, to be used for example in phleboclysis, may consist of an indipendent unit insertable along the adduction tube below the outflow point of the flask. In another version, the device may be an integral part of the flask itself. In both cases, the device according to the invention gives the phleboclysis the function of automatic cut-off which it otherwise lacks.

When the device of the present invention consists of an indipendent unit, an arrow engraved on its outer surface shows the direction in which the liquid must flow for the device to function properly, and thus imparts an immediate and unequivocal instruction for correct mounting.

When the device is incorporated in the defluxing flask itself, it is placed at the latter's outflow end. Since this makes the flask asymetric, its correct position cannot be mistaken, and no external marking is necessary.

The device of the present invention is made of material compatible with the nature of the liquids that are to flow through it. Good results have been obtained with semi-transparent poliethylene, for example.

The moving cut-off and/or sealing element according to the invention may be opaque to specific radiations. In fact, in the version incorporating an alarm signal generator, the cut-off and/or sealing element also functions as the diaphragm of the optical system that generates the signal for an alarm apparatus.

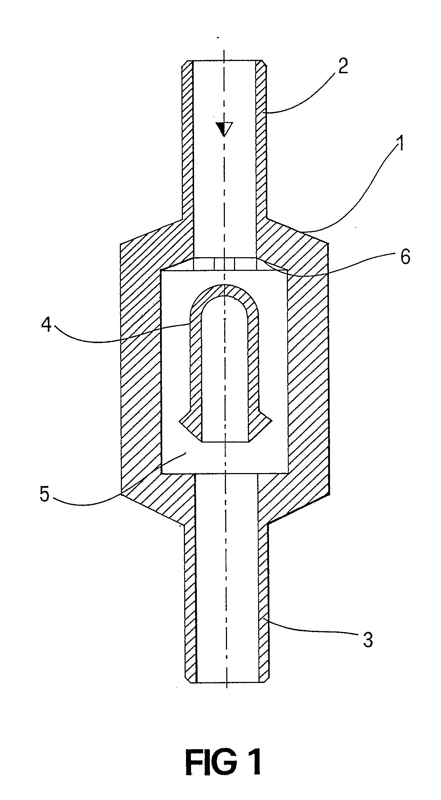

The foregoing is a general description of the invention. Its objects, characteristics, functioning and advantages will now be described in more detail, with reference to the sole figure attached hereto (Figure 1).

Figure 1 is a longitudinal section of an embodiment of the invention comprising an indipendent unit with a cylindrical central chamber and a bell-shaped cut-off element.

With reference to figure 1, the device 1 is inserted by means of neck 2 and neck 3 into a phleboclysis tube intended to transfer a liquid, by force of gravity, from a flask (not shown) positioned above neck 2 to an intravenous needle (not shown) located at the other end of the tube connected to neck 3.

When the device is inserted along the tube line, the force of gravity tends to pull the bell 4 towards the closure positions, which would prevent the liquid from reaching the intravenous needle.

However, if the operator slightly tilts the device (10-20°) the bell shifts out of the closure position and comes to rest along the side wall of the chamber; the liquid can then flow freely into the lower tube until it is completely filled.

As the level of the liquid rises further until the central chamber 5 is full, the bell 4, pushed by the air bubble trapped inside it, rises through the chamber and finally comes to rest at the upper end thereof.

When the bell is in this position, the liquid can still flow into the central chamber 5 along the radial grooves 6 at its upper end.

When the flask is empty and the liquid flowing down through the tube has drained out of the central chamber 5 as well, the bell 4 comes to rest in the sealing position and remains anchored there by the suction effect produced by the column of liquid below.

If the device is to be re-used for another phleboclysis, it needs only to be inclined 10 to 20° as before, while the tube below is simultaneously squeezed slightly between the fingertips. The slight pressure so applied to the liquid is sufficient to dislodge the bell from its sealing position, and the filling process proceeds again as described above.

The chamber 5 of the device according to the invention as described and exemplified herein above may also take the form of two interconnected half-units which can be non-distructively disassembled.

The present invention can be used in combination with all types of flow regulator. Good results have been obtained with a flow regulator consisting of a box, preferably in metal, without bases, with a tetralobate, substantially X-shaped section. The metallic box without bases, in working conditions, surrounds a portion of the flexible effluent tube. The application of a very low compression force on two opposed sides allows their approach while simultaneously the two other opposed sides move away from each other.

Due to the characteristics of material used, the above mentioned deformations are permanent (but also reversible), producing a change on an elastic tube section from the circular form to the elliptic one, thus reducing the inside tube section.

## Claims

1. A device, for preventing the complete draining of liquid from a higher-positioned vessel into its deflux tube, comprising essentially: a chamber, optionally made of two half-units sealed together that can be non-distructively disassembled, having two necks, substantially circular in section, on its opposite ends and internal grooving at the orifice of the upper neck; and a freely moving cut-off and/or sealing element located inside the central part of said chamber, made of material such as to allow it to float in the liquid intended to flow into the device, and shaped so as to lodge in the orifice of either the upper or the lower neck.

2. The device according to claim 1, in which a LED and a photodiode or a photoresistor are incorporated in the upper portion of the central part.

3. The device according to claim 1 or 2, in which the cut-off and/or sealing element is a hollow sphere whose diameter is greater than that of the section of the two necks located at the opposite ends of the chamber.

4. The device according to claim 1 or 2, in which the cut-off and/or sealing element is an upright bell whose dimensions, with respect to those of the chamber, are such that it cannot turn upsidedown inside it.

5. The device according to claim 4, in which the bottom of the bell flairs outwards in the form of a ring whose outer edge slopes downwards.

6. The device according to the preceding claims, made of a flexible and transparent material compatible with the liquids intended to pass through it.

7. The device according to claim 6, made of semi-transparent polyethilene.

8. The device according to claims 2 to 7, in which the cut-off and/or sealing element is made of material opaque to LED radiation.

9. A liquid container whose deflux tube is fitted with the device as per claims 1 to 8 either as an integral part of the container or an indipendent unit to be connected to it.

10. A phleboclysis flask whose deflux tube is fitted with the device as per claims 1 to 8, either as an integral part of the container or as an indipendent unit to be connected to it.

FIG 1